# EUROPÄISCHE PATENTANMELDUNG

(11) **EP 1 700 609 A1**
(43) Veröffentlichungstag der Anmeldung: **13.09.2006**
(21) Anmeldenummer: 05026953.9
(22) Anmeldetag: 09.12.2005
(51) Int. Cl.: A61L 11/00, A01K 1/00

(54) **Einweichanlage und Verfahren zur Einweichung eines Stalles**

(30) Priorität: 13.12.2004 DE 102004060107
(71) Anmelder: Meiner, Wolfgang, 32699 Exertal (DE)
(72) Erfinder: Meiner, Wolfgang, 32699 Exertal (DE)
(74) Vertreter: Schuster, Müller & Partner

(57) **Zusammenfassung**

Es werden eine Einweichanlage (1) und ein Verfahren zur Einweichung eines Stalles mittels einer mobilen Einweichanlage (1) vorgeschlagen, wobei eine Steuerungseinheit (10) die Bewegung der mobilen Einweichanlage (1) steuert, so dass vor dem oder während des Einweichvorganges keine Arbeitskraft gebunden wird, obwohl die erfindungsgemäße Einweichanlage (1) nicht stationär im Stall installiert ist.

## Beschreibung

### Stand der Technik

Die Erfindung geht aus von einer Einweichanlage, nach der Gattung des Anspruchs 1, und von einem Verfahren zur Einweichung eines Stalles, nach der Gattung des Anspruchs 16.

Einweichanlagen dienen neben dem Kühlen von Ställen insbesondere zum Einweichen von Verunreinigungen und zur Desinfektion des Stalles und erhöhen somit die Betriebshygiene. Die Reinigung des Stalles erfolgt hierbei in der Regel unmittelbar nach dem Ausstallen des letzten Tieres, damit die Verunreinigungen (Schmutz, Kot, Futterreste, odgl.) nach Möglichkeit noch feucht sind. Die Verunreinigungen werden durch das durch die Nebeldüsen der Einweichanlage ausgebrachte Wasser durchdrungen und lassen sich bei der anschließenden Reinigung mittels beispielsweise eines Hochdruckreinigers leichter entfernen.

Zum Stand der Technik gehören stationäre Einweichanlagen. Vorteilhaft ist bei stationäre Einweichanlagen die Arbeitszeitersparnis zu beurteilen. Allerdings haben sie den Nachteil, dass sie in jedem Stall installiert sein müssen, teuer sind und eine geringe Akzeptanz bei den Landwirten haben, da sie eine zusätzliche und möglicherweise störanfällige Technik in dem Stall bedeutet. Außerdem investieren Landwirte, die Ställe nur für eine bestimmte Zeit pachten, ungern in eine derartige Einweichanlage. Kostengünstiger ist daher eine mobile Einweichanlage, die, im jeweiligen Stall immer wieder auf und ab gebaut wird, so dass sie durch den Landwirt, flexibel in mehreren Ställen eingesetzt werden kann. Da aber Landwirte in der Regel das zeitaufwändige Einweichen des Stalles nicht zu ihren Lieblingsaufgaben zählen, sind auch mobile Einweichanlagen nachteilig zu bewerten.

### Die Erfindung und ihre Vorteile

Die erfindungsgemäße Einweichanlage, mit dem kennzeichnenden Merkmal des Anspruchs 1, und das erfindungsgemäße Verfahren zur Einweichung eines Stalles, mit dem kennzeichnenden Merkmal des Anspruchs 16, haben demgegenüber den Vorteil, dass eine Steuerungseinheit die Bewegung der mobilen erfindungsgemäßen Einweichanlage steuert, so dass während des Einweichvorganges keine Arbeitskraft gebunden wird, obwohl die erfindungsgemäße Einweichanlage nicht stationär im Stall installiert ist.

Nach einer vorteilhaften Ausgestaltung der erfindungsgemäßen Einweichanlage ist mindestens ein Sensor für einen Fahrtrichtungswechsel vorhanden.

Nach einer diesbezüglichen vorteilhaften Ausgestaltung der erfindungsgemäßen Einweichanlage ist der Sensor mechanisch oder elektronisch (z.B. Ultraschall, Infrarot).

Nach einer zusätzlichen vorteilhaften Ausgestaltung der erfindungsgemäßen Einweichanlage ist der Sensor stirnseitig an der erfindungsgemäßen Einweichanlage angeordnet.

Nach einer zusätzlichen vorteilhaften Ausgestaltung der erfindungsgemäßen Einweichanlage ist mindestens ein seitlich angeordnetes Tastgerät, eine Führung für ein Seil odgl. und/oder ein GPS-Leitsystem vorhanden.

Nach einer diesbezüglichen vorteilhaften Ausgestaltung der erfindungsgemäßen Einweichanlage ist das Tastgerät ein seitliches Leitrad, ein ultraschallgesteuertes Gerät oder ein Puffer.

Nach einer zusätzlichen vorteilhaften Ausgestaltung der erfindungsgemäßen Einweichanlage dient als Leitungssystem mindestens ein Rohrausleger.

Nach einer diesbezüglichen vorteilhaften Ausgestaltung der erfindungsgemäßen Einweichanlage ist der Rohrausleger verstellbar.

Nach einer zusätzlichen vorteilhaften Ausgestaltung der erfmdungsgemäßen Einweichanlage ist der Rohrausleger in Fahrtrichtung gesehen in seiner Arbeitsposition senkrecht zur Fahrtrichtung angeordnet und/ oder nach hinten schwenkbar,

Nach einer diesbezüglichen vorteilhaften Ausgestaltung der erfindungsgemäßen Einweichanlage unterbricht ein Ventil bei Auslenkung des Rohrauslegers aus der Arbeitsposition die Wasserzufuhr zu der Düse.

Nach einer zusätzlichen vorteilhaften Ausgestaltung der erfindungsgemäßen Einweichanlage stoppt ein Drucksensor bei Unterbrechung der Wasserzufuhr den Vortrieb der erfindungsgemäßen Einweichanlage.

Nach einer zusätzlichen vorteilhaften Ausgestaltung der erfindungsgemäßen Einweichanlage weist sie zur Aufnahme einer Applikationslösung und/oder von Wasser mindestens einen Behälter auf.

Nach einer diesbezüglichen vorteilhaften Ausgestaltung der erfindungsgemäßen Einweichanlage erfolgt die Dosierung der Applikationslösung durch eine Pumpe oder durch Ansaugung durch den Wasserstrahl erfolgt.

Nach einer zusätzlichen vorteilhaften Ausgestaltung der erfindungsgemäßen Einweichanlage ist die Applikationslösung ein Medikament, ein Desinfektionsmittel und/oder ein Reinigungsmittel.

Nach einer zusätzlichen vorteilhaften Ausgestaltung der erfindungsgemäßen Einweichanlage ist an dem Wasserversorgungsanschluß ein Versorgungsschlauch anschließbar, wobei der Ausbringdruck an der Düse vom Wasserdruck abhängig ist.

Nach einer zusätzlichen vorteilhaften Ausgestaltung der erfindungsgemäßen Einweichanlage ist über die Steuerungseinheit Sprühpausen, Sprühintervalle und/oder eine Zudosierung der Applikationslösung vorgebbar.

Nach einer zusätzlichen vorteilhaften Ausgestaltung der erfindungsgemäßen Einweichanlage ermöglicht eine Pumpe eine Erhöhung des Wasserdruckes.

Nach einer vorteilhaften Ausgestaltung des erfindungsgemäßen Verfahrens zur Einweichung eines Stalles mittels einer mobilen erfindungsgemäßen Einweichanlage führt die in Betrieb genommene erfindungsgemäße Einweichanlage die Einweichung des Stalles über die mögliche Fahrstrecke selbsttätig durch.

Nach einer zusätzlichen vorteilhaften Ausgestaltung des erfindungsgemäßen Verfahrens liegt die Fahrstrecke der erfindungsgemäßen Einweichanlage in dem Gang zwischen oder neben der Aufstallung oder in der Aufstallung. Denkbar ist auch, dass sich die erfindungsgemäße Einweichanlage an Pfeilern oder Pfosten orientiert oder auf freier Fläche oder in großen Buchten eingesetzt wird.
Nach einer zusätzlichen vorteilhaften Ausgestaltung des erfindungsgemäßen Verfahrens wird ein Festfahren der erfindungsgemäßen Einweichanlage durch die Tastgeräte (Leiträder (14), Puffer) verhindert. Kommt z.B. ein Leitrad der erfindungsgemäßen Einweichanlage mit einer Wand (Buchten-, Stallwand, odgl.) in Berührung, so wird die erfindungsgemäße Einweichanlage über das Leitrad an der Wand entlang geführt.

Nach einer zusätzlichen vorteilhaften Ausgestaltung des erfindungsgemäßen Verfahrens bewirkt ein Hindernis im Bereich des Leitungssystems eine Auslenkung des Leitungssystems.

Nach einer zusätzlichen vorteilhaften Ausgestaltung des erfindungsgemäßen Verfahrens bewirkt ein stirnseitiges im Bereich des Sensors befindliches Hindernis einen Fahrtrichtungswechsel.

Nach einer diesbezüglichen vorteilhaften Ausgestaltung des erfindungsgemäßen Verfahrens wird der Fahrtrichtungswechsel nach einer durch die Steuereinheit vorgebbaren Pause durchgeführt.

Weitere Vorteile und vorteilhafte Ausgestaltungen der Erfindung sind der nachfolgenden Beschreibung, der Zeichnung und den Ansprüchen entnehmbar.

### Zeichnung

Ein Ausführungsbeispiel des Gegenstandes der Erfindung sind in der Zeichnung dargestellt und werden im Folgenden näher erläutert.
Es zeigen:
- Fig. 1: eine schematische Seitendarstellung der erfindungsgemäßen Einweichanlage und
- Fig. 2: eine schematische Frontdarstellung der erFirrdungsgemäßen Einweichanlage.

### Beschreibung des Ausführungsbeispiels

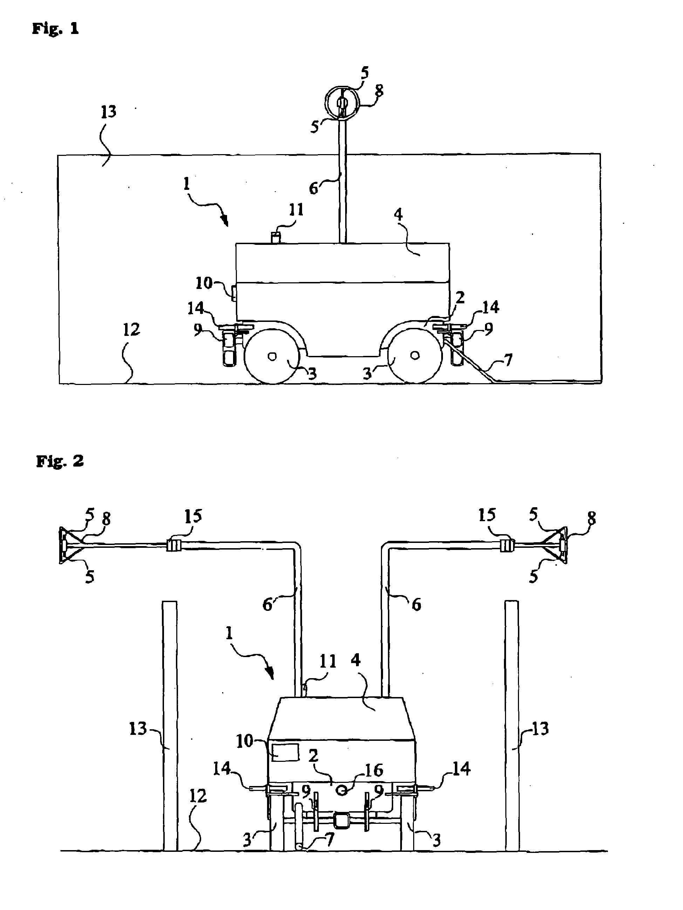

Fig. 1 zeigt eine schematische Seitendarstellung der erfindungsgemäßen Einweichanlage 1, die variabler in unterschiedlichsten Ställen einsetzbar ist, kein Auf- und Abbauen von Leitungen nötig macht, keine Montage erfordert, eine Halbierung der Reinigungszeit gegenüber nicht eingeweichten Ställen bewirkt und die Stallung durch reduzierten Hochdruckreinigereinsatz schont. Diese besteht aus einem Chassis 2, das vorzugsweise für den Transport, um Gewicht zu sparen, aus Aluminium gefertigt ist, und an dem Räder 3, die vorteilhafterweise große Gummiräder sind, um Unebenheiten und Verschmutzungen des Bodens leicht zu überwinden, angeordnet sind, einer Haube 4, die aus Gründen der Haltbarkeit aus Edelstahl gefertigt ist, und aus einem mit Düsen 5 (Rotations-, Prallkörperdüse, odgl.) bestückten Leitungssystem in Form von Rohrausleger 6. An der erfindungsgemäßen Einweichanlage 1 ist ein Wasserschlauch 7 angeschlossen. Die Ausbringung des Wassers über die Düsen 5, die vorzugsweise durch Edelstahlbügel 8 geschützt sind, erfolgt in diesem Ausführungsbeispiel ohne Wasserpumpe alleine durch den gegebenen Wasserdruck. An dem Chassis 2 sind für den Transport ausziehbare Handgriffe 9 angeordnet. Die Steuerung der erfindungsgemäßen Einweichanlage 1 erfolgt über die Steuereinheit 10. Durch diese sind Sprühpausen, Sprühintervalle und/oder eine Zudosierung (Wasserstrahlpumpe) der Applikationslösung, die sich befüllbar über einen Einfüllstutzen 11 in einem nicht dargestellten Behälter befindet vorgebbar.

Fig. 2 zeigt eine schematische Frontdarstellung der erfindungsgemäßen Einweichanlage 1. In Betrieb gesetzt, fährt die erfindungsgemäße Einweichanlage 1, die aufgrund einer geringen Breite (z.B. 50 cm) in nahezu jeden Gang 12 paßt, angetrieben durch einen nicht dargestellten Motor (z.B. akkubetriebener Elektromotor) selbsttätig den Gang 12 zwischen den Buchten entlang. Die Fahrgeschwindigkeit liegt bei ca. 0,5 km/h. Durch seitlich angeordnete Leiträder 14 wird ein Festfahren an den Buchtenwänden 13 vermieden, da sich die erfindungsgemäße Einweichanlage 1 bei Berührung eines Leitrades 14 mit einer Buchtenwand 13 an dieser "entlanghangelt". Die Rohrausleger 6 ragen während des Betriebes über die Buchtenwände 13 in die Bucht hinein und sind mittels eines Teleskops 15 an die Tiefe der Bucht anpassbar. Stößt ein Rohrausleger 6 an ein Hindernis z.B. eine Zuleitung für eine Tränke oder eine Futterleitung, so schwenkt er entgegen der Fahrtrichtung zur Seite. Während des Schwenkvorganges wird die Wasserzufuhr automatisch unterbrochen. Eine Feder bewirkt das Zurückschwenken des Rohrauslegers 6 in seine Arbeitsposition nachdem er das Hindernis passiert hat, wodurch der Sprühvorgang fortgesetzt wird. Ein jeweils stirnseitig angeordneter Sensor 16 ermittelt das Ende des Ganges 12. Dadurch stoppt die Wasserzufuhr und die erfindungsgemäße Einweichanlage 1 bleibt stehen, um einen Fahrtrichtungswechsel vorzunehmen. Nach einer event. vorgegebenen Sprühpause nimmt die erfindungsgemäße Einweichanlage 1 ihre Fahrt in entgegengesetzter Richtung auf. Dadurch fährt die erfindungsgemäße Einweichanlage 1 in der vorgegebenen Zeit in dem Gang 12 immer hin und her. Je nach Stallgröße dauert der selbständige Einweichrhythmus, der variabel einstellbar ist, fünf bis zwanzig Stunden, wobei bei sehr langen Ställen vorteilhafterweise keine Sprühpause zwischen den einzelnen Fahrtrichtungswechseln vorgesehen ist. Mit einem nicht dargestellten Kugelhahn lässt sich bei entsprechender Düseneinrichtung einfach von einer Einweich- auf eine Kühlfunktion umschalten. Durch eine große Auswahl an Düsen 5 läßt sich die erfindungsgemäße Einweichanlage 1 in jedem Stalltyp einsetzen. Zusätzlich kann durch die Wahl der Düsen 5 der zu befeuchtende Bereich (Boden, Wände und/oder Decken) festgelegt werden. Zudem ist die erfindungsgemäße Einweichanlage 1 flexibel aufgebaut, so dass sie das Einweichen auch unterschiedlich großer Stallabteile ermöglicht.

Alle hier dargestellten Merkmale können sowohl einzeln als auch in beliebiger Kombination miteinander erfindungswesentlich sein.

### Bezugszahlenliste

- 1: Einweichanlage
- 2: Chassis
- 3: Rad
- 4: Haube
- 5: Düse
- 6: Rohrausleger
- 7: Wasserschlauch
- 8: Edelstahlbügel
- 9: Handgriff
- 10: Steuereinheit
- 11: Einfüllstutzen
- 12: Gang
- 13: Buchtenwand
- 14: Leitrad
- 15: Teleskop
- 16: Sensor

## Patentansprüche

1. Einweichanlage (1) zur Einweichung von Stallflächen,
- mit einem durch Räder (3) oder Raupen abgestützten Rahmen,
- mit einem Antrieb,
- mit einem Wasserversorgungsanschluß,
- mit einem Leitungssystem und
- mit mindestens einer an diesem Leitungssystem angeordneten Düse (5),
**dadurch gekennzeichnet,**
**dass** eine Steuerungseinheit (10) die Bewegung der Einweichanlage (1) steuert.

2. Einweichanlage (1), nach Anspruch 1,
**dadurch gekennzeichnet,**
**dass** mindestens ein Sensor (16) für einen Fahrtrichtungswechsel vorhanden ist.

3. Einweichanlage (1), nach Anspruch 2,
**dadurch gekennzeichnet,**
**dass** der Sensor (16) mechanisch oder elektronisch ist.

4. Einweichanlage (1), nach Anspruch 2 oder Anspruch 3,
**dadurch gekennzeichnet,**
**dass** der Sensor (16) stirnseitig an der Einweichanlage (1) angeordnet ist.

5. Einweichanlage (1), nach einem der vorhergehenden Ansprüche,
**dadurch gekennzeichnet,**
**dass** mindestens ein seitlich angeordnetes Tastgerät, eine Führung für ein Seil odgl. und/oder ein GPS-Leitsystem vorhanden ist.

6. Einweichanlage (1), nach Anspruch 5,
**dadurch gekennzeichnet,**
**dass** das Tastgerät ein seitliches Leitrad (14), ein ultraschallgesteuertes Gerät oder ein Puffer ist.

7. Einweichanlage (1), nach einem der vorhergehenden Ansprüche,
**dadurch gekennzeichnet,**
**dass** als Leitungssystem mindestens ein Rohrausleger (6) dient.

8. Einweichanlage (1), nach Anspruch 7,
**dadurch gekennzeichnet,**
**dass** der Rohrausleger (6) verstellbar ist.

9. Einweichanlage (1), nach Anspruch 7 oder Anspruch 8,
**dadurch gekennzeichnet,**
**dass** der Rohrausleger (6) in Fahrtrichtung gesehen in seiner Arbeitsposition senkrecht zur Fahrtrichtung angeordnet ist und/oder nach hinten schwenkbar ist.

10. Einweichanlage (1), nach Anspruch 9,
**dadurch gekennzeichnet,**
**dass** ein Ventil bei Auslenkung des Rohrauslegers (6) aus der Arbeitsposition die Wasserzufuhr zu der Düse (5) unterbricht.

11. Einweichanlage (1), nach einem der vorhergehenden Ansprüche,
**dadurch gekennzeichnet,**
**dass** ein Drucksensor bei Unterbrechung der Wasserzufuhr den Vortrieb der Einweichanlage (1) stoppt.

12. Einweichanlage (1), nach einem der vorhergehenden Ansprüche,
**dadurch gekennzeichnet,**
**dass** sic zur Aufnahme einer Applikationslösung und/oder von Wasser mindestens einen Behälter aufweist.

13. Einweichanlage (1), nach Anspruch 12,
**dadurch gekennzeichnet,**
**dass** die Dosierung der Applikationslösung durch eine Pumpe oder durch Ansaugung durch den Wasserstrahl erfolgt.

14. Einweichanlage (1), nach Anspruch 12 oder Anspruch 13,
**dadurch gekennzeichnet,**
**dass** die Applikationslösung ein Medikament, ein Desinfektionsmittel und/oder ein Reinigungsmittel ist.

15. Einweichanlage (1), nach einem der vorhergehenden Ansprüche,
**dadurch gekennzeichnet,**
**dass** an dem Wasserversorgungsanschluß ein Versorgungsschlauch anschließbar ist, wobei der Ausbringdruck an der Düse (5) vom Wasserdruck abhängig ist.

16. Einweichanlage (1), nach einem der vorhergehenden Ansprüche,
**dadurch gekennzeichnet,**
**dass** über die Steuerungseinheit (10) Sprühpausen, Sprühintervalle und/oder eine Zudosierung der Applikationslösung vorgebbar ist.

17. Einweichanlage (1), nach einem der vorhergehenden Ansprüche,
**dadurch gekennzeichnet,**
**dass** eine Pumpe eine Erhöhung des Wasserdruckes ermöglicht.

18. Verfahren zur Einweichung eines Stalles mittels einer mobilen Einweichanlage (1),
**dadurch gekennzeichnet,**
**dass** eine Einweichanlage (1) nach einem der Ansprüche 1 bis 17 verwendet wird.

19. Verfahren, nach Anspruch 18,
**dadurch gekennzeichnet,**
**dass** die in Betrieb genommene Einweichanlage (1) die Einweichung des Stalles über die mögliche Fahrstrecke selbsttätig durchführt.

20. Verfahren, nach Anspruch 18 oder Anspruch 19,
**dadurch gekennzeichnet,**
**dass** die Fahrstrecke der Einweichanlage (1) in dem Gang (12) zwischen oder neben der Aufstallung oder in der Aufstallung liegt.

21. Verfahren, nach einem der Ansprüche 18 bis 20,
**dadurch gekennzeichnet,**
**dass** ein Festfahren der Einweichanlage (1) durch die Tastgeräte (Leiträder (14), Puffer) verhindert wird.

22. Verfahren, nach einem der Ansprüche 18 bis 21,
**dadurch gekennzeichnet,**
**dass** ein Hindernis im Bereich des Leitungssystems eine Auslenkung des Leitungssystems bewirkt.

23. Verfahren, nach einem der Ansprüche 18 bis 22,
**dadurch gekennzeichnet,**
**dass** ein stirnseitiges im Bereich des Sensors (16) befindliches Hindernis einen Fahrtrichtungswechsel bewirkt.

24. Verfahren, nach Anspruch 23,
**dadurch gekennzeichnet,**
**dass** der Fahrtrichtungswechsel nach einer durch die Steuereinheit (10) vorgebbaren Pause durchgeführt wird.
